# EUROPEAN PATENT SPECIFICATION

(11) **EP 4 197 468 B1**
(45) Date of publication and mention of the grant of the patent: **29.10.2025**
(21) Application number: 22214041.0
(22) Date of filing: 16.12.2022
(51) Int. Cl.: A61B 18/12, A61B 18/16

(54) **IRREVERSIBLE ELECTROPORATION RETURN ELECTRODE AND SYSTEM**
IRREVERSIBLE ELEKTROPORATIONSRÜCKELEKTRODE UND SYSTEM
ÉLECTRODE ET SYSTÈME DE RETOUR D'ÉLECTROPORATION IRRÉVERSIBLE

(30) Priority: 17.12.2021 US 202117555220
(43) Date of publication of application: 21.06.2023
(73) Proprietor: BIOSENSE WEBSTER (ISRAEL) LTD., 2066717 Yokneam (IL)
(72) Inventor: GOVARI, Assaf, 2066717 Yokneam (IL); ALTMANN, Andres Claudio, 2066717 Yokneam (IL)
(74) Representative: Carpmaels & Ransford LLP

(56) References cited:
- CN-A- 113 100 916
- US-A1- 2011 202 055
- US-A1- 2013 158 543
- US-A1- 2021 161 592

## Description

### TECHNICAL FIELD

The present disclosure relates generally to irreversible electroporation (IRE) systems, and particularly to methods and systems for electrodes used therewith.

### BACKGROUND OF THE DISCLOSURE

Irreversible electroporation (IRE) is a soft tissue ablation technique that applies short pulses of strong electrical fields to create permanent and hence lethal nanopores in the cell membrane, thus disrupting the cellular homeostasis (internal physical and chemical conditions). Cell death following IRE results from apoptosis (programmed cell death) and not necrosis (cell injury, which results in the destruction of a cell through the action of its own enzymes) as in other thermal or radiation based ablation techniques.

Unipolar irreversible electroporation (IRE) systems typically operate at high currents, for example, approximately 30 Amperes and higher. Some IRE procedures use unipolar IRE pulses, which are returned to the IRE generator via a return electrode, e.g., an electrode coupled to a backpatch. These pulses are typically balanced, and as such, have zero DC Current.

US 2013/158543 A1 describes an electrosurgical return electrode. The return electrode includes first and second flexible conductive material layers and a material layer disposed between the first and second conductive material layers. The material layer is transitionable between a solid state and a non-solid state, the material layer is also configured to melt upon an increase in temperature beyond a predetermined threshold, thereby increasing conductivity between the first and second conductive material layer.

US 2011/202055 A1 describes a method for determining a temperature and/or a temperature change at a neutral electrode having a contacting agent layer. The method comprises determining at least one impedance value of the contacting agent layer and calculating a temperature change and/or a temperature at the neutral electrode, at least on the basis of the impedance value.

The present disclosure will be more fully understood from the following detailed description of the examples thereof, taken together with the drawings in which:

### BRIEF DESCRIPTION OF THE DRAWINGS

Fig. 1 is an illustration of example IRE system in use with a patient;
FIG. 2 is a diagram that schematically illustrates an example system in use with the disclosed subject matter, in accordance with an example of the present disclosure;
Fig. 3A is a schematic view of a single electrode patch, in accordance with an example of the disclosed subject matter;
Figs. 3B-1 and 3B-2 are schematic views of dual electrode patches, in accordance with other examples of the disclosed subject matter;

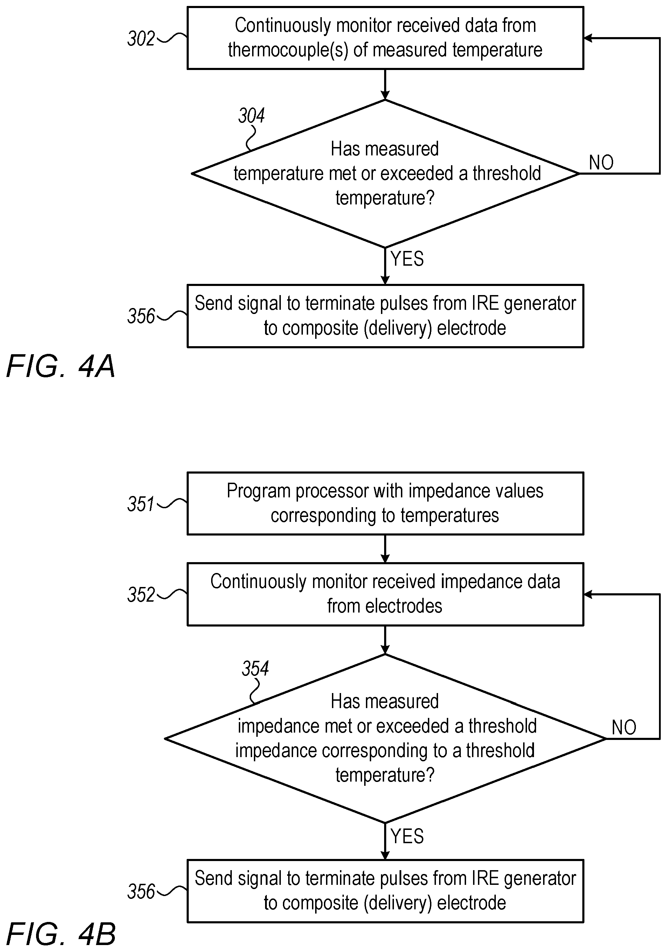

Fig. 4A is a flow diagram of an example process based on temperatures detected by a thermocouple for shutting off the disclosed system, in accordance with an example of the disclosed subject matter; and
Fig. 4B is a flow diagram of an example process based on impedance measurements between electrodes for shutting off the disclosed system, in accordance with an example of the disclosed subject matter.

### DETAILED DESCRIPTION OF EXAMPLES

The present disclosed subject matter provides a body surface electrode, such as a patch, and, for example, a backpatch, which automatically detects detachment from the skin and/or proximity thereto, within a predetermined limit (hereinafter referred to as "detachment"), and hence, the electrode coming out of contact with and/or proximity to the skin. Once the detachment is detected, the disclosed system for example, automatically and/or instantaneously, terminates pulse delivery, to a pulse delivery electrode, from an IRE generator, which generates the delivered pulses. By automatically and/or instantaneously terminating pulse delivery to the pulse delivery electrode, at the time backpatch electrode detachment is detected, overheating of body tissue, possibly resulting in damage to the body tissue, is minimized, if any.

The disclosed system, by its automatic termination of pulses to the delivery electrode, is faster and more accurate that periodic checking of the attachment of the backpatch to the skin by human attendants, or by the patient feeling any detachment. Visual inspections performed by human attendants, may not see or otherwise misjudge the presence of any detachments. Additionally, the patient may suffer from nerve and/or skin conditions, and may not feel any detachment of the backpatch.

### OVERVIEW

Some IRE schemes are unipolar, in the sense that pulses are applied, for example, between two unipolar electrodes, one of a catheter, and the other attached to the patient, typically at the back, closest to the catheter. The electrode(s Irreversible electroporation (IRE) is a predominantly non-thermal process, which causes an increase of the tissue temperature by, at most, a few degrees for a few milliseconds. IRE differs from RF (radio frequency) ablation, which typically raises the tissue temperature by between 20 and 70°C and destroys cells through heating. Although a system for IRE is described below, the systems and methods disclosed herein are equally applicable to both IRE and RF ablation, with RF systems using catheters modified for RF generated current pulses.

Some IRE schemes are unipolar, in the sense that pulses are applied, for example, between two unipolar electrodes, one of a catheter, and the other attached to the patient, typically at the back, closest to the catheter. The electrode(s) are typically on a body surface electrode, for example, in the form of a back patch, which, for example, adhesively attaches to the skin of the patient.

As stated above, two different sensing methods are generally used for sensing physiological signals, unipolar sensing and bipolar sensing. Unipolar sensing has one electrode in contact with or in close proximity to tissue and another electrode, and another electrode outside of the tissue that is being sensed.

In order for the IRE-pulses to generate the required nanopores in tissue, the field strength E of the pulses must exceed a tissue-dependent threshold Eₜₕ. Thus, for example, for heart cells the threshold is approximately 500 V/cm, whereas for bone it is 3000 V/cm. These differences in threshold field strengths enable IRE to be applied selectively to different tissues.

When performing unipolar IRE, it is important that the body-surface electrode (e.g., backpatch) be attached reliably to the patient's body. Otherwise, the detachment of the body surface electrode results in tissue damage caused by the pulses overheating the tissue, as the body surface electrode is no longer properly returning the current (associated with the pulses) to the IRE generator.

Examples of the disclosed subject matter that are described herein provide improved body-surface electrodes for use in unipolar IRE procedures, and associated methods. The disclosed techniques provide for reliably and continuously monitoring and determining, by a processor, computer or the like, whether a body surface electrode is properly positioned, e.g., attached, with respect to a patient's body, to receive pulses (e.g., current) from a pulse delivery electrode and subsequently return the current to the IRE generator. Should the body surface electrode become improperly positioned, the detection thereof by the processor is instantaneous, and accordingly, pulse delivery is instantaneously terminated. This instantaneous termination is, for example, typically performed by the processor signaling the IRE generator to stop generating pulses, or signaling a switching box to open a line. In both cases, pulses are immediately stopped from reaching the pulse delivery electrode. As a result of this instantaneous pulse termination, any tissue damage from overheating, caused by pulses, resulting from the detached or now improperly positioned body surface electrode, is minimized and typically avoided completely.

### SYSTEM DESCRIPTION

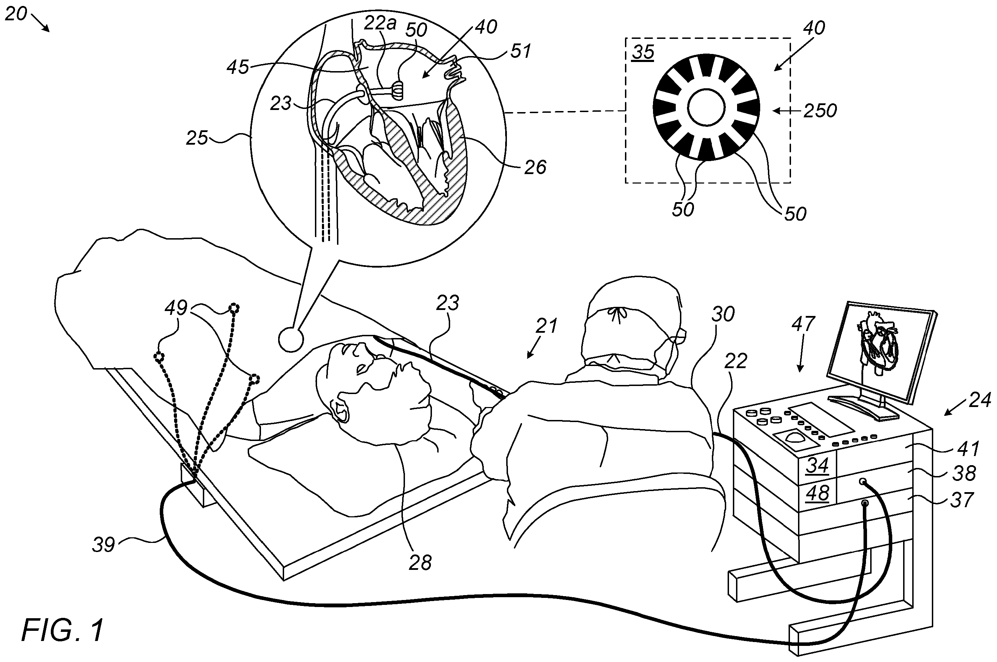

Fig. 1 is a schematic, pictorial illustration of a catheter-based irreversible electroporation (IRE) system 20, in accordance with an example of the disclosed subject matter. The system 20 comprises a catheter 21, wherein a shaft 22 of the catheter is inserted by a physician 30 through the vascular system of a patient 28 through a sheath 23. The physician then navigates a distal end 22a of shaft 22 to a target location inside a heart 26 of the patient.

Once the distal end 22a of the shaft 22 has reached the target location, physician 30 retracts sheath 23 and expands balloon 40, typically by pumping saline into balloon 40. Physician 30 then manipulates shaft 22 such that electrodes 50 disposed on the balloon 40 catheter engage an interior wall of a PV ostium 51 to apply high-voltage PFA pulses via electrodes 50 to ostium 51 tissue.

As seen in inset 25, distal end 22a is fitted with an expandable balloon 40 comprising multiple equidistant IRE electrodes 50. Due to the flattened shape of the distal portion of balloon 40, the distance between adjacent electrodes 50 remains approximately constant even where electrodes 50 cover the distal portion. Balloon 40 configuration therefore allows more effective electroporation (e.g., with approximately uniform electric field strength) between adjacent electrodes 50, as in inset 35.

Certain aspects of inflatable balloons are addressed, for example, in U.S. Patent Application Serial No. 16/993,092, filed August 13, 2020, titled "Applying Bipolar Ablation Energy Between Shorted Electrode Groups," which is assigned to the assignee of the present patent application.

In the example described herein, which is, for example, a unipolar IRE system, catheter 21 may be used for any suitable diagnostic and/or therapeutic purpose, such as electrophysiological sensing and/or the aforementioned IRE isolation of PV ostium 51 tissue in left atrium 45 of heart 26.

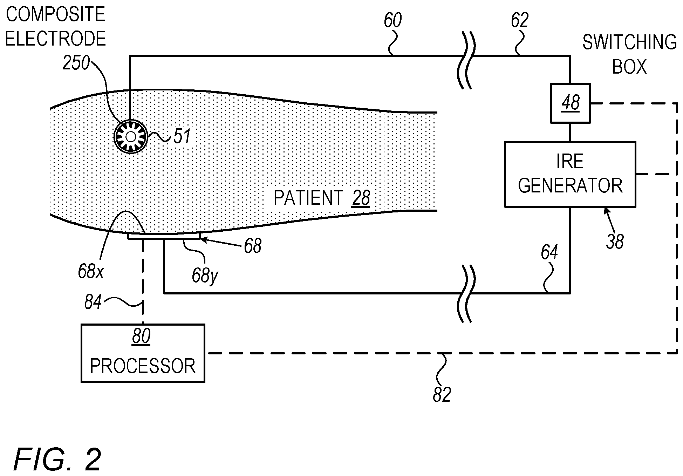

The proximal end of catheter 21 is connected to switching assembly 48 comprised in console 24, with circuitry for creating an effective composite electrode 250 by short-circuiting electrodes 50 one to the other (e.g., using switches of assembly 48). Electrodes 50 are connected to the switching assembly 48 PFA by electrical wiring (shown in Fig. 2) running in shaft 22 of catheter 21. Console 24 further comprises a PFA pulse generator 38, to which assembly 48 is connected, where generator 38 is configured to apply the PFA pulses between composite electrode 250 and a skin patch electrode (shown in Fig. 2), which serves as a return electrode.

An IRE pulse generator similar to PFA pulse generator 38 is described in U.S. Patent Application 16/701,989, filed December 3, 2019, titled "Pulse Generator for Irreversible Electroporation," which is assigned to the assignee of the present patent application. The pulses generated by the pulse generator 38 are balanced, i.e., they have zero DC.

A memory 34 of console 24 stores IRE protocols comprising PFA pulse parameters, such as peak-to-peak voltage and pulse width, as described for Fig. 1.

Console 24 comprises a processor 41, typically a general-purpose computer, with suitable front end and interface circuits 37 for receiving signals from catheter 21 and from external electrodes 49, which are typically placed around the chest of patient 28. For this purpose, processor 41 is connected to external electrodes 49 by wires running through a cable 39.

During a procedure, system 20 can track the respective locations of electrodes 50 inside heart 26 using the Active Current Location (ACL) method, provided by Biosense-Webster (Irvine, California), which is described in U.S. Patent 8,456, 182.

In some examples, physician 30 can modify, from a user interface 47, any of the parameters of the unipolar IRE protocol used with composite electrode 250. User interface 47 may comprise any suitable type of input device, e.g., a keyboard, a mouse, or a trackball, among others.

Processor 41 is typically programmed in software to carry out the functions necessary to perform IRE procedures, including those procedures performed by unipolar IRE. The software may be downloaded to the computer in electronic form, over a network, for example, or it may, alternatively or additionally, be provided and/or stored on non-transitory tangible media, such as magnetic, optical, or electronic memory.

In particular, processor 41 runs a dedicated protocol as disclosed herein, which enables processor 41 to perform the disclosed steps, as further described below.

### USING UNIPOLAR CONFIGURATION FOR IRE

Fig. 2 shows a schematic, pictorial illustration the example system 20 in operation on a patient 28. For example, the patient 28 is a human or other mammalian subject. The irreversible electroporation (IRE) catheter 40 of Fig. 1 is ablating ostium 51 of a pulmonary vein (PV) with pulse trains of unipolar pulsed-field ablation (PFA) pulses of current (the pulses of current also known as pulsed current or current pulses, and these terms are used interchangeably herein), for example, as described in U.S. Patent Application Serial No. 17/073,467, titled: "Using Unipolar Configuration For Irreversible Electroporation", which is assigned to the assignee of the present patent.

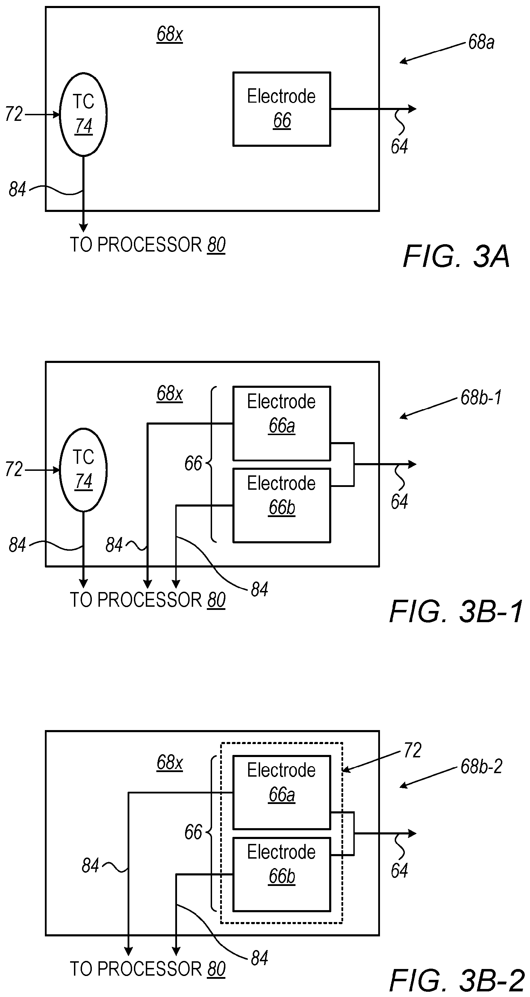

The composite electrode 250, also known as a pulse delivery electrode, is in contact with ostium 51 over an entire circumference of the ostium. The composite electrode is connected via a cable 60 to switching assembly (of one or more switches) 48, where the separate wires of cable 60 that connect to electrodes 50 are short-circuited one with the other in the switching assembly 48, to create an effective composite or pulse delivery electrode 250. A single conductor 62 connects switching assembly 48 to one end of PFA generator 38, also known as an IRE generator or generator, and to the cable 60. The other lead of PFA generator 38 is connected by a line 64 to an electrode 66 (Figs. 3A, 3B-1 and 3B-2) on a body surface electrode, for example, shown as a back patch 68. The line 64 facilitates return current from the electrode(s) 66 to the IRE (PFA) generator 38. The IRE generator 38, switching box 48 (if part of the system 20), lines 60/62, composite electrode 250, backpatch 68 via the return electrode 66, and, return line 64, form an electrical circuit for pulse delivery to and return from the patient 28.

The back patch 68 is attached to skin of the patient 28, for example, at an adhesive side 68x of the back patch 68. The outer side 68y of the back patch 66 is exposed to the ambient environment. As long as the backpatch 68 is well attached to the skin, chances for overheating in the body are minimal, if any. However, if the backpatch 68 detaches from the skin, even partly, there may be local overheating which may cause burning in the body, and tissue damage from the burning. As noted above, implementing unipolar PFA using electrode 250 requires dedicated IRE protocols with suitable PFA pulses to be applied to the patient 28. In some examples, a provided PFA protocol divides (partitions) the PFA pulse delivery of a selected protocol into multiple pulse trains ("pulse bursts") with pauses between the pulse trains, for example, as described in U.S. Patent Application 16/701,989, filed December 3, 2019, titled "Pulse Generator for Irreversible Electroporation", which is assigned to the assignee of the present patent application. The pauses permit muscle relaxation, if any contraction occurs.

The electrode 66 (Figs. 3A, 3B-1, 3B-2), which is typically one, but may also be a more than one electrode, is, for example, positioned on or otherwise coupled to, the adhesive or inner side 68x of the patch. The patch 68 electrode 66 is typically aligned with the composite (pulse delivery) electrode 250, and is placed proximately to, and typically into contact with the skin of patient 28, at the patch 68 adheres to the skin, along the back 28x of the patient 28. The adherence is, for example, by adhesives, so that the patch 68 is either self-attaching, or with other mechanical or chemical fasteners.

The patch 68, also includes a temperature measuring device 72, temperature detector, or temperature sensor (collectively referred to hereinafter as a temperature measuring device), positioned on or otherwise coupled to the patch 68 on the adhesive side 68x of patch 68. The temperature measuring device 72, for example, outputs one or more signals indicative of the measured, detected or sensed temperature (collectively referred to herein as the measured temperature). The temperature measuring device 72 is, for example, a thermocouple (TC) 74 (as shown in Figs. 3A and 3B-1), the dual electrodes 66a, 66b, by being analyzed for electrical impedance between them also serve as a temperature measuring device, represented by the broken line box 72, as shown, for example, in Fig. 3B-2, and described below. While one temperature measuring device 72 is shown, the temperature measuring device may include multiple temperature measuring devices, such as multiple thermocouples 74, and the like.

The IRE generator 38, may operate, for example, at approximately: a frequency of 1 Mega Hertz (MHz), a voltage of 2 kilo Volts (kV), and, an impedance of 30 Amperes (A). IRE generator 38, is, for example, operable at approximately 5 nF (nano Farads).

Alternately, a radiofrequency (RF) generator may be used to generate the pulses (of current) in a manner similar to that for the PFA (IRE) generator 38, detailed in this section of this document. In this case, the IRE catheter 40 would be replaced by an RF catheter, to accommodate and transmit RF pulses from the RF Generator.

One or more processors, represented, for example, by the processor 80 are electronically connected to the switching box 48 and/or the IRE generator 38, and, the temperature measuring device 72, by wired or wireless connections 82, 84, shown by broken lines. The processor 80 obtains the outputted temperature signal(s), and analyzes the temperature represented by the signal(s), against a threshold temperature, programmed into the processor 80. Should the temperature represented by the outputted temperature signal(s), at least meet or exceed the threshold temperature, the processor 80 signals: a) the switching box 48, to open the connection 62, prohibiting pulses from traveling over the line 60/62 and reaching the composite electrode 250, or, b) the IRE generator 38 to shut off. These two signaling actions cause termination, inhibition and/or suppression the delivery and/or application of IRE pulses, for example, from the IRE generator 38 to the composite electrode 250. In doing so, tissue damage from overheating is minimized or avoided.

The processor 80 communicates with the temperature measuring device 72, which includes, for example, one or more thermocouples (TC) 74, and typically one thermocouple, on the patch 68 (represented by patches 68a and 68b-1), as shown in Figs. 3A and 3B-1, or spaced apart electrodes 66a, 66b in the broken line box on the patch 68 (represented by the patch 68b-2) in Fig. 3B-2, via wired or wireless connections 84. The processor 80 receives the measured temperature, e.g., data corresponding to the measured temperature, and analyzes this received temperature data, for example, by comparing the associated measured temperature against a preprogrammed, preset or predetermined threshold temperature, at which the IRE generator 38 needs to be shut off, to terminate the pulses, such that the pulses do not reach (are not delivered to) the composite (delivery) electrode 250, or, the pulses from the IRE generator 38 to the composite electrode 250 are terminated by a switch change in the switching box 48, opening the lines 60/62. Terminating the pulses occurs when at least the threshold temperature is detected and is determined by the processor 80, which causes, for example, by signaling, the instantaneous turning off of the IRE generator 38, or changing of the switching in the switching box 48. This detection and signaling is instantaneous, and as such, minimizes or avoids tissue damage, from any extra and unintended IRE pulses.

The patch 68 is, for example, one of the patches 68a, 68b-1 and 68b-2, as shown in Figs. 3A, 3B-1 and 3B-2, respectively.

Turning to the patch 68a of Fig. 3A, on the adhesive side 68x of the patch 68a, which contacts the skin, is a single electrode 66 and one or more thermocouples (TC) 74. The thermocouple 74, for example, operates as the temperature measuring device 72, and is in electronic communication with the processor 80, via the wired or wireless link 84.

The processor 80 obtains the measured temperature from the thermocouple 74. For example, the thermocouple 74 either sends signals indicative of measured temperature to the processor 80, either, continuously or at various time intervals including randomly, and, either automatically, or when triggered by the processor 80. Alternately, the processor 80 monitors the thermocouple 74, for example, monitoring the data of measured temperature, as measured by the thermocouple(s) 74, which is outputted by the thermocouple 74. The monitoring is, for example, continuous, and may be constant or in intervals, equally spaced apart in time, or random.

When the patch 68a with the electrode 66 separates from the skin, the processor 80 obtains the temperature measured by the thermocouple 74. In a detachment, for example, the temperature has lowered, due to exposure of the thermocouple to the ambient environment. This temperature (e.g., in the form of temperature data) is outputted by the thermocouple 74, and transmitted to, or detected by, the processor 80. Should the detected temperature (of the temperature data) meet or exceed a predetermined or threshold temperature, as determined by the processor 80, the processor 80 signals: 1) the switching box 48 to change the switch to open the line 60/62, so as to stop or otherwise terminate pulses from the IRE generator 38 from reaching the composite electrode 250, and/or 2) the IRE generator 38, to terminate pulse generation, the aforementioned actions terminating the pulses, i.e., pulse delivery, from the IRE generator 38 to the composite electrode 250. As a result, any potential overheating and/or burning of tissue, resulting in tissue damage, is avoided or minimized.

Alternately, as shown in the example of Fig. 3B-1, on the patch 68b-1, the electrode 66 is divided into a plurality of electrodes or sub-electrodes, for example, two or dual electrodes 66a, 66b, spaced apart from each other. The spacing is such that an electrical impedance between the electrodes 66a, 66b can be measured. There is one or more thermocouples, for example, one thermocouple 74. The thermocouple 74, for example, operates as a temperature measuring device, and is in electronic communication with the processor 80, via the connection 84. Should the measured temperature meet or exceed a predetermined or threshold temperature, as determined by the processor 80, the processor 80 signals: 1) the switching box 48 to change the switch to open the line 60/62, so as to stop or otherwise terminate pulses from the IRE generator 38 from reaching the composite electrode 250, and/or 2) the IRE generator 38 to terminate pulse generation, the aforementioned actions terminating the pulses, i.e., pulse delivery, from the IRE generator 38 to the composite electrode 250. As a result, any potential overheating and/or burning of tissue, resulting in tissue damage, is avoided or minimized.

The thermocouple 74 may operate as the sole temperature measuring device, or in parallel with, the sub-electrodes 66a, 66b. Alternately, the sub-electrodes 66a, 66b may operate as the temperature detector, so as to be another temperature measuring device in addition to the thermocouple 74, or without the thermocouple 74 functioning as a temperature measuring device. When operating as a temperature measuring device, the electrode 66 is such that its spaced apart electrodes 66a, 66b link to the processor 80 by the wired or wireless links 84.

The electrodes 66a, 66b are spaced apart at a predetermined distance on the adhesive side 68x of the patch 68b-1. The spacing is such that local impedance between the electrodes 66a, 66b, through the skin is measured, and the impedance measurement (e.g., as data) is sent to the processor 80. The measured impedance corresponds to a measured temperature and, for example, is obtained by the processor 80 as temperature data. For example, a temperature rise in the skin lowers the impedance, so that a preset decrease in impedance, as programmed into the processor 80, is used by the processor 80, as the threshold or preset temperature. Should the measured temperature (of the temperature data) meet or exceed a predetermined or threshold temperature, as determined by the processor 80, the processor 80 signals: 1) the switching box 48 to change the switch to open the line 60/62, so as to stop or otherwise terminate pulses from the IRE generator 38 from reaching the composite electrode 250, and/or 2) the IRE generator 38, to terminate pulse generation, the aforementioned actions terminating the pulses, i.e., pulse delivery, from the IRE generator 38 to the composite electrode 250. As a result, any potential overheating and/or burning of tissue, resulting in tissue damage, is avoided or minimized.

Fig. 3B-2 shows another example of the patch 68b-2, including an electrode 66 formed of two electrodes 66a, 66b (functioning as a temperature sensor 72 in the broken line box as well as electrodes), from which local impedance across the skin is measured, and transmitted to the processor 80 for analysis (the analysis as detailed for Fig. 3B-1 above). The electrodes 66a, 66b and processor 80 operate similarly to that described above for the patch 68b-1, and as shown in Fig. 3B-1, for signaling the switching box 48 and/or the IRE generator 38 to stop or otherwise terminate pulses (e.g., pulses delivered from the IRE generator 38) from reaching to the composite electrode 250, as detailed above, for the systems using the patches 68a and 68b-1.

Fig. 4A is a flow diagram of an example process performed by the processor 80 that schematically illustrates a method for using thermocouples 74 to detect a detachment of the patch 68 (represented, for example, by patches 68a and 68b-1 in FIGs. 3A and 3B-1, respectively) from the skin of a patient 28, and the termination of pulses from the IRE generator 38 from reaching the composite electrode 250, in accordance with examples of the present disclosure. The process is, for example, performed automatically and in real time, and may be performed, including being repeated, for as long as desired.

At block 302, the process begins, as the processor 80, for example, monitors the data of measured temperature, as measured by the thermocouple(s) 74, which is outputted and transmitted to, and received by, or obtained by, the processor 80. The monitoring is, for example, continuous, and may be constant or in intervals, equally spaced apart in time, or random.

The process moves to block 304, where the processor 80 determines whether the measured temperature has met or exceeded a threshold temperature. For example, at the threshold temperature, the skin is considered to be overheating and the internal tissue is subject to being damaged. For a patch for which the electrode(s) is partially removed or otherwise partially separated from contact with the skin, this threshold temperature is, for example, approximately 45C, for at above 47.5C skin can sustain first-degree burns; and above 55C skin can sustain second-degree burns.

If no at block 304, the process returns to block 302, from where it resumes, as discussed above. If yes at block 304, the temperature threshold has been met or exceeded, which triggers the processor 80 to cause termination of the pulse(s) from the IRE generator 38 to the composite electrode 250. This is achieved, for example, by stopping pulse generation and/or not allowing generated pulses to reach the composite electrode 250. For example, the processor 80 sends a signal(s) to or signals: 1) the switching box 48 to change the switch to open the line 60/62, so as to stop or otherwise terminate pulses from the IRE generator 38 from reaching the composite electrode 250, and/or 2) the IRE generator 38, to terminate pulse generation, the aforementioned actions terminating the pulses, i.e., pulse delivery, from the IRE generator 38 to the composite electrode 250. As a result, any potential overheating and/or burning of tissue, resulting in tissue damage, is avoided or minimized.

Fig. 4B is a flow diagram of an example process performed by the processor 80 that schematically illustrates a method for detecting a detachment of the patch 68 (represented, for example, by patch 68b-2 in Fig. 3B-2) from the skin of a patient 28, based on detecting electrical impedance through the skin between electrodes 66a, 66b, in accordance with examples of the present disclosure. The process is, for example, performed automatically and in real time, and may be performed, including being repeated, for as long as desired.

At block 351, the process begins. Here, the processor 80 is programmed with electrical impedance values corresponding to temperatures, and one or more threshold impedance values corresponding to one or more threshold temperatures, indicative of overheating of the skin and internal tissue damage. Once the requisite threshold temperature is determined by the processor 80 (having obtained the electrical impedance values corresponding to temperatures), to have been reached, the processor 80 will generate signals for terminating (e.g., shutting off, stopping, inhibiting) pulse delivery from the IRE generator 38 to the composite electrode 250.

At block 352, as the processor 80, for example, monitors the impedance values, e.g., data, outputted from the electrodes 66a, 66b received or obtained from the electrodes 66a, 66b. The monitoring is, for example, continuous, and may be constant or in intervals, equally spaced apart in time, or random.

The process moves to block 354, where the processor 80 determines whether the measured impedance has met or exceeded a threshold impedance, corresponding to a threshold temperature, for example, where the skin is considered to be overheating and the internal tissue is subject to being damaged. For a patch for which the electrodes are partially removed or otherwise partially separated from contact with the skin, this threshold temperature is, for example, approximately 45C (as detailed above, 47.5C skin can sustain first-degree burns, and above 55C skin can sustain second-degree burns).

If no at block 354, the process returns to block 352, from where it resumes, as discussed above. If yes at block 354, the impedance is such that the temperature threshold has been met or exceeded, which triggers the processor 80 to cause termination of the pulse(s) from the IRE generator 38 to the composite electrode 250. This is achieved, for example, by stopping pulse generation and/or not allowing generated pulses to reach the composite electrode 250. For example, the processor 80 sends a signal(s) to or signals: 1) the switching box 48 to change the switch to open the line 60/62, so as to stop or otherwise terminate pulses from the IRE generator 38 from reaching the composite electrode 250, and/or 2) the IRE generator 38, to terminate pulse generation, the aforementioned actions terminating the pulses, i.e., pulse delivery, from the IRE generator 38 to the composite electrode 250. As a result, any potential overheating and/or burning of tissue, resulting in tissue damage, is avoided or minimized.

Typically, the processor 80 comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

Although the examples described herein mainly address electrode contacts with a patient's skin for a backpatch, the methods and systems described herein can also be used in other applications, such as in contact detection, between electrodes and surfaces.

## Claims

1. A patch detachment detection system (20) for controlling delivery of pulses of current from a generator (38), the system comprising:
an Irreversible Electroporation generator; and
a body surface electrode (68) comprising:
at least one electrode (66) for coupling with a pulse delivery electrode (250) in communication with the Irreversible Electroporation generator (38), the at least one electrode (66) being adapted to, in use, be in communication with the skin of a patient, and the at least one electrode (66) for communication with the Irreversible Electroporation generator (38) to return current to the generator (38); and
at least one temperature measuring device (72); and
a processor (80) in communication with the at least one temperature measuring device (72), the processor (80) programmed to:
obtain data corresponding to measured temperature from the at least one temperature measuring device (72);
analyze the obtained data to determine whether the measured temperature at least meets a threshold temperature; and
transmit a signal to terminate pulse delivery from the Irreversible Electroporation generator (38) to the pulse delivery electrode (250), if the threshold temperature is met.

2. The patch detachment detection system (20) of claim 1, wherein the body surface electrode (68) includes a patch for adhering to the skin of the patient.

3. The patch detachment detection system (20) of claim 1 or claim 2, wherein the at least one temperature measuring device (72) comprises a thermocouple (74).

4. The patch detachment detection system (20) of any preceding claim, wherein the at least one electrode (66) comprises two electrodes (66a, 66b) spaced apart a predetermined distance from each other along the body surface electrode (68) to measure electrical impedance which is included in the data corresponding to the measured temperature, and, the two electrodes forming the at least one temperature measuring device (72).

5. The patch detachment detection system (20) of any preceding claim, wherein the processor (80) is programmed to transmit the signal to the Irreversible Electroporation generator (38) to terminate the delivery of the pulses of current.

6. The patch detachment detection system (20) of any preceding claim, additionally comprising:
a switch (48) intermediate the Irreversible Electroporation generator (38) and the pulse delivery electrode (250) for moving between a first position, where pulses are moving between the Irreversible Electroporation generator (38) and the pulse delivery electrode (250), and a second position, where the pulses are prohibited from moving between the Irreversible Electroporation generator (38) and the pulse delivery electrode (250), and
the processor (80) is programmed to transmit the signal to the switch (48) to move to the second position, to terminate the delivery of the pulses of current.

7. The patch detachment detection system (20) of claim **6,** wherein the Irreversible Electroporation generator (38), the switch (48), the pulse delivery electrode (250), and the at least one electrode (66) of the body surface electrode (68) form an electrical circuit.

8. The patch detachment detection system (20) of any of claims 1 to 6, wherein the Irreversible Electroporation generator (38), the pulse delivery electrode (250), and the at least one electrode (66) of the body surface electrode (68) form an electrical circuit.

## Patentansprüche

1. Pflasterablösungserkennungssystem (20) zum Steuern einer Abgabe von Stromimpulsen von einem Generator (38), das System umfassend:
einen Generator für irreversible Elektroporation; und
eine Körperoberflächenelektrode (68), umfassend:
mindestens eine Elektrode (66) zum Koppeln mit einer Impulsabgabeelektrode (250) in Austausch mit dem Generator (38) für irreversible Elektroporation, wobei die mindestens eine Elektrode (66) geeignet ist, um, in Verwendung, mit der Haut eines Patienten in Austausch zu stehen, und die mindestens eine Elektrode (66) für Austausch mit dem Generator (38) für irreversible Elektroporation Strom an den Generator (38) zurückzugeben soll; und
mindestens eine Temperaturmessvorrichtung (72); und
einen Prozessor (80) in Austausch mit der mindestens einen Temperaturmessvorrichtung (72), wobei der Prozessor (80) programmiert ist zum:
Erhalten von Daten, die gemessener Temperatur von der mindestens einen Temperaturmessvorrichtung (72) entsprechen;
Analysieren der erhaltenen Daten, um zu bestimmen, ob die gemessene Temperatur mindestens eine Schwellentemperatur erreicht; und
Übertragen eines Signals, um die Impulsabgabe von dem Generator (38) für irreversible Elektroporation an die Impulsabgabeelektrode (250) zu beenden, falls die Schwellentemperatur erreicht ist.

2. Pflasterablösungserkennungssystem (20) nach Anspruch 1, wobei die Körperoberflächenelektrode (68) ein Pflaster zum Aufkleben auf die Haut des Patienten einschließt.

3. Pflasterablösungserkennungssystem (20) nach Anspruch 1 oder 2, wobei die mindestens eine Temperaturmessvorrichtung (72) ein Thermoelement (74) umfasst.

4. Pflasterablösungserkennungssystem (20) nach einem der vorstehenden Ansprüche, wobei die mindestens eine Elektrode (66) zwei Elektroden (66a, 66b) umfasst, die in einem zuvor bestimmten Abstand voneinander entlang der Körperoberflächenelektrode (68) beabstandet sind, um die elektrische Impedanz zu messen, die in den Daten eingeschlossen ist, die der gemessenen Temperatur entsprechen, und wobei die zwei Elektroden die mindestens eine Temperaturmessvorrichtung (72) ausbilden.

5. Pflasterablösungserkennungssystem (20) nach einem der vorstehenden Ansprüche, wobei der Prozessor (80) programmiert ist, um das Signal an den Generator (38) für irreversible Elektroporation zu übertragen, um die Abgabe der Stromimpulse zu beenden.

6. Pflasterablösungserkennungssystem (20) nach einem der vorstehenden Ansprüche, zusätzlich umfassend:
einen Schalter (48) zwischen dem Generator (38) für irreversible Elektroporation und der Impulsabgabeelektrode (250) zum Bewegen zwischen einer ersten Position, in der sich Impulse zwischen dem Generator (38) für irreversible Elektroporation und der Impulsabgabeelektrode (250) bewegen, und einer zweiten Position, in der die Impulse daran gehindert werden, sich zwischen dem Generator (38) für irreversible Elektroporation und der Impulsabgabeelektrode (250) zu bewegen, und
der Prozessor (80) programmiert ist, um das Signal an den Schalter (48) zu übertragen, sich in die zweite Position zu bewegen, um die Abgabe der Stromimpulse zu beenden.

7. Pflasterablösungserkennungssystem (20) nach Anspruch 6, wobei der Generator (38) für irreversible Elektroporation, der Schalter (48), die Impulsabgabeelektrode (250) und die mindestens eine Elektrode (66) der Körperoberflächenelektrode (68) einen elektrischen Schaltkreis ausbilden.

8. Pflasterablösungserkennungssystem (20) nach einem der Ansprüche 1 bis 6, wobei der Generator (38) für irreversible Elektroporation, die Impulsabgabeelektrode (250) und die mindestens eine Elektrode (66) der Körperoberflächenelektrode (68) einen elektrischen Schaltkreis ausbilden.

## Revendications

1. Système de détection de détachement de patch (20) permettant de commander la délivrance d'impulsions de courant à partir d'un générateur (38), le système comprenant :
un générateur d'électroporation irréversible ; et
une électrode de surface corporelle (68) comprenant :
au moins une électrode (66) destinée à s'accoupler à une électrode de délivrance d'impulsions (250) en communication avec le générateur d'électroporation irréversible (38), l'au moins une électrode (66) étant conçue, en cours d'utilisation, pour être en communication avec la peau d'un patient, et l'au moins une électrode (66) étant destinée à communiquer avec le générateur d'électroporation irréversible (38) pour renvoyer du courant au générateur (38) ; et
au moins un dispositif de mesure de température (72) ; et
un processeur (80) en communication avec l'au moins un dispositif de mesure de température (72), le processeur (80) étant programmé pour :
obtenir des données correspondant à une température mesurée provenant de l'au moins un dispositif de mesure de température (72) ;
analyser les données obtenues pour déterminer si la température mesurée respecte au moins une température seuil ; et
transmettre un signal pour terminer la délivrance d'impulsions par le générateur d'électroporation irréversible (38) à l'électrode de délivrance d'impulsions (250), si la température seuil est respectée.

2. Système de détection de détachement de patch (20) selon la revendication 1, dans lequel l'électrode de surface corporelle (68) comporte un patch destiné à adhérer à la peau du patient.

3. Système de détection de détachement de patch (20) selon la revendication 1 ou la revendication 2, dans lequel l'au moins un dispositif de mesure de température (72) comprend un thermocouple (74).

4. Système de détection de détachement de patch (20) selon l'une quelconque revendication précédente, dans lequel l'au moins une électrode (66) comprend deux électrodes (66a, 66b) espacées d'une distance prédéterminée l'une de l'autre le long de l'électrode de surface corporelle (68) pour mesurer une impédance électrique qui est incluse dans les données correspondant à la température mesurée, et, les deux électrodes formant l'au moins un dispositif de mesure de température (72).

5. Système de détection de détachement de patch (20) selon l'une quelconque revendication précédente, dans lequel le processeur (80) est programmé pour transmettre le signal au générateur d'électroporation irréversible (38) pour terminer la délivrance des impulsions de courant.

6. Système de détection de détachement de patch (20) selon l'une quelconque revendication précédente, comprenant en outre :
un commutateur (48) entre le générateur d'électroporation irréversible (38) et l'électrode de délivrance d'impulsions (250) destiné à se déplacer entre une première position, où des impulsions se déplacent entre le générateur d'électroporation irréversible (38) et l'électrode de délivrance d'impulsions (250), et une seconde position, où il est interdit aux impulsions de se déplacer entre le générateur d'électroporation irréversible (38) et l'électrode de délivrance d'impulsions (250), et
le processeur (80) est programmé pour transmettre le signal au commutateur (48) pour se déplacer à la seconde position, pour terminer la délivrance des impulsions de courant.

7. Système de détection de détachement de patch (20) selon la revendication 6, dans lequel le générateur d'électroporation irréversible (38), le commutateur (48), l'électrode de délivrance d'impulsions (250) et l'au moins une électrode (66) de l'électrode de surface corporelle (68) forment un circuit électrique.

8. Système de détection de détachement de patch (20) selon l'une quelconque des revendications 1 à 6, dans lequel le générateur d'électroporation irréversible (38), l'électrode de délivrance d'impulsions (250) et l'au moins une électrode (66) de l'électrode de surface corporelle (68) forment un circuit électrique.
